# FASCICULE DE BREVET EUROPEEN

(11) **EP 1 165 837 B1**
(45) Date de publication et mention de la délivrance du brevet: **12.09.2007**
(21) Numéro de dépôt: 00917157.0
(22) Date de dépôt: 07.04.2000
(51) Int. Cl.: C12Q 1/68

(54) **PROCEDE DE DETECTION DE MARQUEURS D`UNE MOLECULE D`ADN COMPRENANT UNE ETAPE D`AMPLIFICATION AD INFINITUM**
VERFAHREN ZUM NACHWEIS VON MARKERN EINES DNS-MOLEKÜLS UNTER VERWENDUNG EINES AD INFINITUM AMPLIFIZIERUNGSSCHRITTES
METHOD FOR DETECTING MARKERS OF A DNA MOLECULE COMPRISING AN AD INFINITUM AMPLIFICATION STEP

(30) Priorité: 08.04.1999 FR 9904388
(43) Date de publication de la demande: 02.01.2002
(73) Titulaire: CENTRE NATIONAL DE LA RECHERCHE SCIENTIFIQUE (CNRS), 75794 Paris Cedex 16 (FR)
(72) Inventeur: GALIBERT, Francis, F-95210 Saint Gratien (FR); CAVALOC, Yvon, 98800 Noumea (NC)
(74) Mandataire: Warcoin, Jacques
(86) Numéro de dépôt international: PCT/FR2000/000891
(87) Numéro de publication internationale: WO 2000/061798

(56) Documents cités:
- WO-A-97/10252
- US-A- 5 731 171
- DEAR P H ET AL: "HAPPY MAPPING: A PROPOSAL FOR LINKAGE MAPPING THE HUMAN GENOME" NUCLEIC ACIDS RESEARCH,GB,OXFORD UNIVERSITY PRESS, SURREY, vol. 17, no. 17, page 6795-6807 XP000371654 ISSN: 0305-1048
- DEAR P H: "HAPPY mapping. In: Genome mapping, Dear P H (Ed.)" 1997 , IRL PRESS , OXFORD, UK XP000863731 page 95 -page 123
- DEAR AND COOK: "HAPPY MAPPING: LINKAGE MAPPING USING A PHYSICAL ANALOGUE OF MEIOSIS" NUCLEIC ACIDS RESEARCH,GB,OXFORD UNIVERSITY PRESS, SURREY, vol. 21, no. 1, page 13-20 XP002088488 ISSN: 0305-1048 cité dans la demande
- DEAR P H ET AL.: "A high-resolution metric HAPPY map of human chromosome 14" GENOMICS, vol. 48, 1998, pages 232-241, XP000865519 cité dans la demande
- GROTHUES D ET AL.: "PCR amplification of megabase DNA with tagged random primers (T-PCR)" NUCLEIC ACID RESEARCH, vol. 21, no. 5, 1993, pages 1321-1322, XP002126258 cité dans la demande
- WALTER G ET AL.: "HAPPY mapping of a YAC reveals alternative haplotypes in the human immunoglobin VH locus" NUCLEIC ACIDS RESEARCH, vol. 21, no. 19, 1993, pages 4524-4529, XP002126259
- CHEUNG V G AND NELSON S F: "Whole genome amplification using a degenerate oligonucleotide primer allows hundreds of genotypes to be performed on less than one nanogram of genomic DNA" PROCEEDINGS OF THE NATIONAL ACADEMY OF SCIENCES USA, vol. 93, 1996, pages 14676-14679, XP002126260 cité dans la demande
- GYAPAY G ET AL.: "A radiation hybrid map of the human genome" HUMAN MOLECULAR GENETICS, vol. 5, no. 3, 1996, pages 339-346, XP002126261 cité dans la demande
- HE S ET AL: "DETECTION OF DNA SEQUENCE POLYMORPHISMS AMONG WHEAT VARIETIES" THEORETICAL AND APPLIED GENETICS,DE,SPRINGER, BERLIN, vol. 84, no. 5/06, page 573-578 XP000672708 ISSN: 0040-5752

## Description

La présente invention concerne un procédé de détection de marqueurs d'une molécule d'ADN du type Happy Mapping qui comprend une étape permettant l'amplification *ad infinitum* de l'ADN de chaque panel. La présente invention concerne également un kit pour mettre en oeuvre ce procédé de cartographie, et l'utilisation des cartes obtenues pour l'identification de gènes conférant un phénotype d'intérêt.

L'émergence de très nombreux projets de séquençage de génomes et notamment du génome humain nécessite immanquablement le développement de nouvelles méthodes rapides et précises de cartographie afin d'effectuer l'assemblage des données brutes issues du séquençage systématique. De plus, si comme la tendance actuelle de procéder par des shotguns de génomes entiers se confirme, il sera nécessaire de disposer de cartes génomiques détaillées.

Plus d'une douzaine de génomes microbiens ont déjà été séquencés entièrement, suite à un shotgun direct de leur génome (http:/www.tigr.org/tdb/mdb/mdb.html). Cependant, ceux-ci présentent des tailles allant de 580 kb à 4 Mb. L'utilisation de cette approche dans le but de séquencer de plus grands génomes tels que le génome humain (3 000 Mb), comme cela a été proposé par Weber et Myers, 1997, et annoncé par Venter et al., 1998 suscite néanmoins certaines interrogations. En effet, la taille importante de ces génomes et la présence de nombreuses séquences répétées rend difficile l'assemblage des résultats du séquençage. Ainsi, il s'avère nécessaire de disposer de cartes génomiques détaillées pour permettre le traitement de ces données.

L'établissement de cartes génomiques détaillées peut être également d'un grand secours pour des études phylogéniques. En effet, les études sur l'évolution des génomes ont clairement monté que l'expression de nombreux gènes dépend de leur localisation dans un certain contexte génétique. Les développements récents de la génomique permettent maintenant d'étudier plus finement l'évolution des génomes en se fondant sur les changements de relation de synténie. Des cartes détaillées, obtenues pour deux espèces, permettent d'évaluer les liaisons génétiques qui se sont maintenues entre ces deux espèces au cours de l'évolution.

Un autre domaine pour lequel l'apport de cartes génétiques détaillées serait d'un intérêt capital est la localisation de QTL (Quantitative Trait Loci). En effet, la plupart des variations au sein d'une population ou entre des races différentes, par exemple, est de nature quantitative. Certaines variations, telles que la taille, le poids des individus, la date de floraison chez les plantes ou bien la quantité de lait produite chez les mammifères ne rentrent pas dans des classes bien définies suivant les proportions Mendeliennes, mais plutôt s'opèrent de manière continue selon un gradient d'un extrême à l'autre. Ces variations, conservées dans la descendance, sont donc transmises génétiquement. Les loci impliqués dans la variation de traits phénotypiques quantitatifs sont appelés QTL. La détection de liaisons entre un QTL et des marqueurs génétiques apportent une méthode robuste pour l'identification de ces QTL. Il est possible de localiser un QTL par la méthode dite `Interval mapping' (Lander et Botstein, 1989) entre deux marqueurs informatifs séparés de plus de 20 cM. Toutefois, un tel intervalle rend l'identification du gène problématique. Aussi, il est tout à fait utile de pouvoir effectuer un zoom sur la région d'intérêt si on dispose d'un grand nombre de marqueurs, et d'effectuer une cartographie fine, afin de localiser précisément le gène recherché.

Une approche de cartographie envisageable est la cartographie par hybrides d'irradiation (RH, pour *Radiation Hybrid mapping*) (Cox *et al.,* 1990 ; Gyapay *et al.,* 1996). Elle consiste à irradier des lignées cellulaires, ce qui provoque des cassures aléatoires chromosomiques. Les différents fragments de chromosomes générés sont alors intégrés dans le génome de cellules de rongeur. Ainsi, il est possible de déterminer la distance séparant deux marqueurs sachant que plus ils sont proches, plus ils auront de chance d'être incorporés au sein d'un même fragment et donc d'être détectés dans une même lignée. Néanmoins, cette approche présente certains désavantages en plus de sa lourdeur dans la mise en place d'un panel d'hybrides d'irradiation. En effet, *(i)* certains loci qui ne se seraient pas clonés ne peuvent être intégrés dans une carte du génome; *(ii)* l'interprétation des résultats peut être confuse dans le cas où les inserts se sont réarrangés ou ligués entre eux ; *(iii)* la présence d'ADN exogène, en l'occurrence celui de la cellule hôte de hamster, oblige bien souvent à écarter un nombre de marqueurs donnant une réponse positive avec cet ADN exogène.

La méthode Happy Mapping, plus souple, permet de contourner ces différents problèmes (Dear et Cook, 1993). Cette méthode permet de reproduire *in vitro* les deux événements analysés par les croisements de la génétique formelle, c'est à dire le crossing-over et la ségrégation. En pratique, le crossing-over est mimé par une cassure aléatoire d'ADN en fragments dont la taille dépend de la cartographie recherchée. Les marqueurs sont alors ségrégés par distribution aléatoire de ces fragments en dépôts de moins d'un équivalent de génome haploïde par aliquot, puis détectés par PCR. Ceux qui sont génétiquement liés tendent à demeurer ensemble dans le même aliquot tandis que ceux qui ne sont pas liés se distribuent de façon aléatoire. Leur ordre et la distance qui les sépare peuvent être déduits de la fréquence de leur co-ségrégation par calcul statistique. Il est important de rappeler ici qu'un panel utilisable pour la cartographie Happy est simple à produire puisqu'il ne requiert que quelques jours voire quelques semaines. En outre, il est adaptable à n'importe quel niveau de résolution, selon la taille des fragments sélectionnés et peut même aboutir au clonage moléculaire des fragments d'intérêt en vue d'un séquençage.

La méthode du Happy Mapping consiste en les étapes suivantes :
a) L'ADN génomique est cassé par irradiation,
b) Environ un équivalent de génome haploïde est ensuite placé dans chaque puits d'une plaque à 96 puits, ce qui correspond environ à 60% de l'ADN génomique de départ (distribution statistique des marqueurs),
c) l'ADN est amplifié par PCR,
d) et les marqueurs sont ensuite détectés.

Cette méthode de cartographie a déjà fait ses preuves pour des génomes aussi variés en taille que le chromosome 14 humain -~100 Mb- (Dear et al., 1998) ou celui d'un protozoaire parasite de l'épithélium intestinal de nombreux mammifères; Cryptosporidium parvum -10 Mb- (Piper et al., 1998). Toutefois, pour ces deux études, aucune méthode satisfaisante d'amplification du panel initial, permettant de cartographier un nombre illimité de marqueurs et toute sorte de marqueurs n'a été décrite. Dans Dear et al., seulement une faible partie de l'ADN total, encadrée par des séquences répétées, a pu être cartographiée. Dans Piper *et al.,* le niveau d'amplification n'était pas suffisant pour permettre la détection directe par PCR des marqueurs. Ces auteurs ont du procéder par PCR nichée afin de visualiser les marqueurs à cartographier. De plus, la méthode d'amplification utilisée ne permet que de cartographier un nombre limité de marqueurs, nécessitant de reconstruire un panel de cartographie pour localiser des marqueurs supplémentaires.

Afin d'être envisageable pour la cartographie Happy, la méthode d'amplification doit répondre aux trois critères suivants :
*(i)* Une quantité d'ADN voisine d'un équivalent de génome haploïde doit être suffisante comme matrice;
*(ii)* l'intégralité de l'information génétique doit être amplifiée;
*(iii)* le panel constitué doit pouvoir être réamplifié à l'infini afin de permettre la cartographie d'un nombre illimité de marqueurs.

Ainsi, le problème consiste à amplifier la totalité de l'ADN dans chaque puits, ladite amplification ne devant pas produire d'artefacts dans la distribution aléatoire des marqueurs. L'objectif est la mise au point d'une approche d'amplification totale, homogène et à l'infini de l'ADN génomique.

La technique PCR classique a évolué pour donner de nombreuses méthodes d'amplification ayant chacune leur spécificité. Par exemple, Il est possible d'amplifier plusieurs séquences simultanément en utilisant plusieurs couples d'amorces dans un même tube réactionnel, Apostolakos et al, (1993). Cependant, le nombre de couples d'amorces dépasse rarement 3. En effet, au-delà, les amplifications perdent leur spécificité. D'autres techniques, dérivées plus ou moins de la PCR, ont été développées : La LCR, la Gap-LCR, l'ERA, la CPR. la SDA, la TAS, la NASBA. Cependant aucune de ces techniques d'amplification ne semble offrir une solution adéquate à l'amplification totale de l'ADN.

La technique T-PCR consiste en une première étape d'amplification à l'aide d'amorces contenant, en leur extrémité 3', des séquences aléatoires reproduisant toutes les combinaisons possibles, et une séquence définie en leur extrémité 5'. Ces oligonucléotides vont dans les conditions opératoires s'apparier de façon aléatoire sur toute la longueur de la séquence et les cycles d'amplification permettront l'incorporation de ladite séquence définie à tous les fragments amplifiés. La seconde étape consiste à amplifier les fragments obtenus à la première étape à l'aide d'une amorce comportant uniquement la séquence définie de l'extrémité 5' des amorces de la première étape. Cette technique a été décrite dans US 5,731,171, et Grothues et al, 1993. Selon les auteurs, ce procédé permet l'amplification de fragments d'ADN de 400 pb mais aussi de génomes pouvant aller jusqu'à 40 mégabases. Pour qu'une technique PCR soit applicable au Happy Mapping, l'amplification doit être générale tout en n'introduisant pas de sélection (biais) dans les portions d'ADN amplifié. Or, ce point n'a été testé que par hybridation, ce qui n'est pas démonstratif. Par ailleurs, US 5,731,171 montre qu'il n'est possible de faire une amplification totale seulement si on dispose au départ d'au moins 17 équivalents d'ADN. A priori, ceci montre que la méthode d'amplification T-PCR ne peut pas être utilisée pour la cartographie par la méthode Happy Mapping puisque par principe on doit amplifier des quantités d'ADN qui ne correspondent qu'à 1 équivalent. En outre, l'étape d'amplification est présentée dans US 5,731,171 en vue d'obtenir des marqueurs et non pour préparer le substrat sur lequel seront positionnés les marqueurs. Le fait que l'inventeur du Happy Mapping lui-même n'ait pas retenu la T-PCR, mais plutôt la NESTED-PCR lors des développements ultérieurs de sa technique démontre que la technique telle qu'elle était décrite et testée par hybridation n'apparaissait pas satisfaisante. La solution trouvée dans le cadre de la présente invention a été d'accommoder la T-PCR au Happy Mapping. La méthodologie mise au point s'avère être avantageuse pour amplifier la totalité de l'ADN dans chaque puits sans introduire d'artefacts. Dès lors, cette méthode d'amplification représente un support technique qui permet au Happy Mapping de prendre toute son ampleur.

Ainsi, la présente invention concerne un procédé de détection de marqueurs d'une molécule d'ADN caractérisé en ce qu'il comprend les étapes consistant à :
a) Casser la molécule d'ADN de sorte à obtenir des fragments d'ADN dont la taille dépend de la résolution choisie,
b) répartir lesdits fragments dans des réceptacles de façon à avoir une quantité d'ADN comprise entre environ 0,5 à 1,5 équivalent génome haploïde d'ADN par réceptacle,
c) amplifier l'ADN contenu dans les réceptacles par une méthode d'amplification comprenant les étapes suivantes : i) Une première amplification à l'aide d'une amorce comprenant de 10 à 30 nucléotides définis en son extrémité 5', et de 5 à 10 nucléotides aléatoires en son extrémité 3', et ii) une deuxième amplification à l'aide d'une amorce comprenant au moins les oligonucléotides définis de l'extrémité 5' de l'amorce utilisée à l'étape i).
d) détecter la présence ou l'absence de marqueurs dans les réceptacles.

De préférence, l'amorce utilisée à l'étape i) comporte 20 nucléotides définis en son extrémité 5', et 6 nucléotides aléatoires en son extrémité 3'. Dans ce cas, l'amorce utilisée à l'étape ii) peut comprendre les 20 nucléotides définis en l'extrémité 5' de l'amorce utilisée à l'étape i).

De manière tout à fait avantageuse, l'amorce utilisée à l'étape i) correspond à la séquence SEQ ID n°1 et l'amorce utilisée à l'étape ii) correspond à la séquence SEQ ID n°2.

Cette méthode d'amplification réside donc en deux étapes complémentaires. La première phase consiste en une amplification avec un seul oligonucléotide tel que décrit ci-dessus, et la réaction est effectuée dans un volume final de 30 à 70 µl par puits de microplaque, de préférence 50µl, avec de 3 à 7 unités, de préférence 5 unités d'AmpliTaq polymérase (Perkin Elmer). Toute polymérase équivalente à l'AmpliTaq peut être utilisée avec de 2 à 6µM d'amorce, de préférence 4µM.

La réaction de PCR peut être réalisée de la manière suivante (@ signifie « à» ou « environ à »): 1x [5 min @95°C] ; 50x [45 sec @92°C, 2 min @37°C, 37°C-55°C 0.1°C/sec. 4 min @55°C]: 15x [45 sec @92°C, 1 min @55°C, 3 min @72°C]; 1x [5 min @72°C]. Bien entendu, tout cycle équivalent peut être mis en oeuvre par exécuter l'invention.

Pour la seconde phase de la réaction, de 1/20^{e} à 1/200^{e}, de préférence 1/50^{e} du produit obtenu lors de la première phase est utilisé comme matrice. La réaction PCR peut être effectuée dans un volume réactionnel final de 5 à 20µl, de préférence 10µl par puits de microplaque. La Taq DNA polymérase (Promega) peut être utilisée avec de 0,5 à 3µM, de préférence 1.5µM d'amorce telle que définie ci-dessus (amorce de l'étape ii)). La réaction PCR peut être effectuée selon le cycle suivant : 1x [2 min @94°C]; 50x [30 sec @92°C, 45 sec @54°C, 3 min @,72°C]; 1x [5 min @72°C].

Bien entendu les paramètres de cette méthode d'amplification peuvent être modifiés ou accommodés selon le cas d'espèce par l'homme du métier.

On entend par « molécule d'ADN » dans le cadre de l'invention, une molécule qui correspond à un génome, un chromosome, ou à un fragments d'un génome ou d'un chromosome. En outre, ladite molécule peut provenir d'un génome ou d'un chromosome qui a éventuellement subi des modification et/ou des traitements.

Un mode de réalisation préféré de l'invention consiste en l'extraction de la molécule d'ADN à partir de cellules encapsulées dans des blocs d'agarose puis lysées afin de libérer ladite molécule intacte. Lesdites cellules peuvent correspondre à n'importe quelle cellule des règnes végétal, animal ou bactérien.

La molécule d'ADN isolée est ensuite cassé par irradiation γ, par digestion enzymatique, notamment par l'action d'endonucléases, telles par exemple les enzymes de restriction, et/ou par action mécanique. Les fragments d'ADN obtenus peuvent être séparés par toute technique de séparation connue de l'homme du métier, notamment par électrophorèse, de préférence par électrophorèse en champs pulsés pour obtenir des fragments de grande taille avant la répartition (étape b) du procédé décrit ci-dessus).

Les plaques de microtitration sont utilisées avantageusement comme réceptacles. Ainsi, les fragments sont répartis dans les 96 puits d'une plaque de microtitration. A cet effet, les fragments sont répartis de sorte à avoir une quantité d'ADN par réceptacle (de préférence par puits) à environ 1 équivalent génome haploïde, soit de l'ordre de 2 pg pour un génome de mammifère par exemple.

Ainsi, le procédé selon l'invention se caractérise également en ce qu'il comprend une étape qui permet d'amplifier l'intégralité de l'information génétique contenu dans les réceptacles.

L'ADN, amplifié dans chaque puits, peut ensuite être distribué de manière à préparer des plaques filles. Dans ce cas, les marqueurs sont détectés dans les puits des plaques filles. Toutefois, la détection des marqueurs peut aussi être effectuée directement dans les puits de la plaque mère mais dans ce cas, seul un nombre limité de marqueurs pourra être analysé.

Les marqueurs, susceptibles d'être présent dans les puits, peuvent être amplifiés à l'aide d'amorces spécifiques avant l'étape de détection. La détection s'effectue habituellement après migration électrophorétique dans un gel approprié à la taille du fragment. La détection peut s'effectuer également à l'aide de sondes spécifiques des marqueurs. Les sondes peuvent être des sondes de captures fixées directement ou indirectement sur un support solide ou des sondes à l'état libre. Une « sonde de capture » est immobilisée ou immobilisable sur un support solide par tout moyen approprié, par exemple par covalence, par adsorption, ou par synthèse directe sur un support solide. Ces techniques sont notamment décrites dans la demande de brevet WO 9210092. Une « sonde de détection » peut être marquée au moyen d'un marqueur choisi par exemple parmi les isotopes radioactifs, des enzymes, en particulier des enzymes susceptibles d'agir sur un substrat chromogène, fluorigène ou luminescent (notamment une peroxydase ou une phosphatase alcaline), des composés chimiques chromophores, des composés chromogènes, fluorigènes ou luminescents, des analogues de base nucléotitiques, et des ligands tels que la biotine. Les méthodes de détection en microplaques 96 puits sont à la portée de l'homme du métier.

Par exemple, le produit d'amplification PCR est dénaturé et hybridé dans un puits de microplaque sur lequel est fixé un oligonucléotide de capture, Running (1990) ou un ADN simple-brin contenant une séquence de capture, Kawai (1993). Une au moins des amorces utilisées dans la PCR est par exemple biotinylée et la détection de l'hybridation est réalisée par addition de streptavidine couplée à une enzyme telle que la peroxydase, puis d'un substrat chromogène de l'enzyme. En utilisant un oligonucléotide de capture fixé sur les puits, une amorce de PCR biotinylée, et un standard interne d'amplification, il a même été possible de réaliser de la PCR quantitative. Berndt (1995).

Un mode préféré de réalisation de la présente invention réside en la détection des marqueurs sur des puces à ADN. Le principe de cette technique consiste à identifier des séquences d'ADN sur la base d'une hybridation moléculaire. La puce porte, greffée sur une surface adéquate, des centaines ou milliers d'oligonucléotides d'intérêt ou produits PCR correspondant aux marqueurs que l'on souhaite cartographier. L'ADN des puits est dénaturé, marqué, puis mis dans des conditions d'hybridation avec la puce. L'avantage des puces réside dans leur capacité à fournir à partir d'un échantillon d'ADN des centaines, voir des milliers d'informations. De plus, le schéma général de l'expérimentation est très simple et rapide.

Un autre aspect de l'invention concerne un kit pour cartographier une molécule d'ADN caractérisé en ce qu'il permet de mettre en oeuvre le procédé selon l'invention. Ce kit peut comprendre notamment une amorce comprenant de 10 à 30 nucléotides définis en son extrémité 5' et de 5 à 10 nucléotides aléatoires en son extrémité 3', et/ou une amorce comprenant au moins les oligonucléotides définis de l'extrémité 5' de l'amorce précédemment évoquée. De préférence, le kit comprend une amorce de la séquence SEQ ID n°1, et/ou une amorce de la séquence SEQ ID n°2. Le kit mentionné ci-dessus est donc utile pour la préparation des panels nécessaires à l'établissement de cartes génomiques ou chromosomiques.

Un autre aspect de l'invention porte sur l'utilisation du procédé selon l'invention pour l'identification de gènes conférant un phénotype d'intérêt (notamment chez les plantes), pour l'identification de gènes responsable de maladies héréditaires, notamment chez les humains, pour l'identification de QTL (Quantitative Trait Loci). Un autre aspect porte sur l'utilisation de cartes selon l'invention pour une aide à la reconstruction de shotguns massifs destiné au séquençage d'une molécule d'ADN.

On se référera aux légendes des figures présentées ci-après pour la suite de la description.

### Légendes

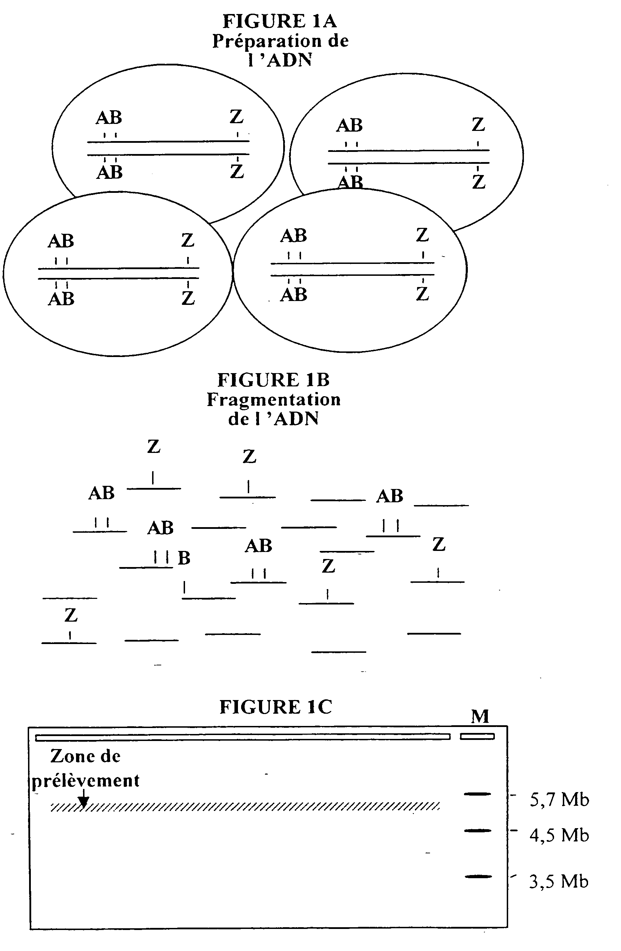

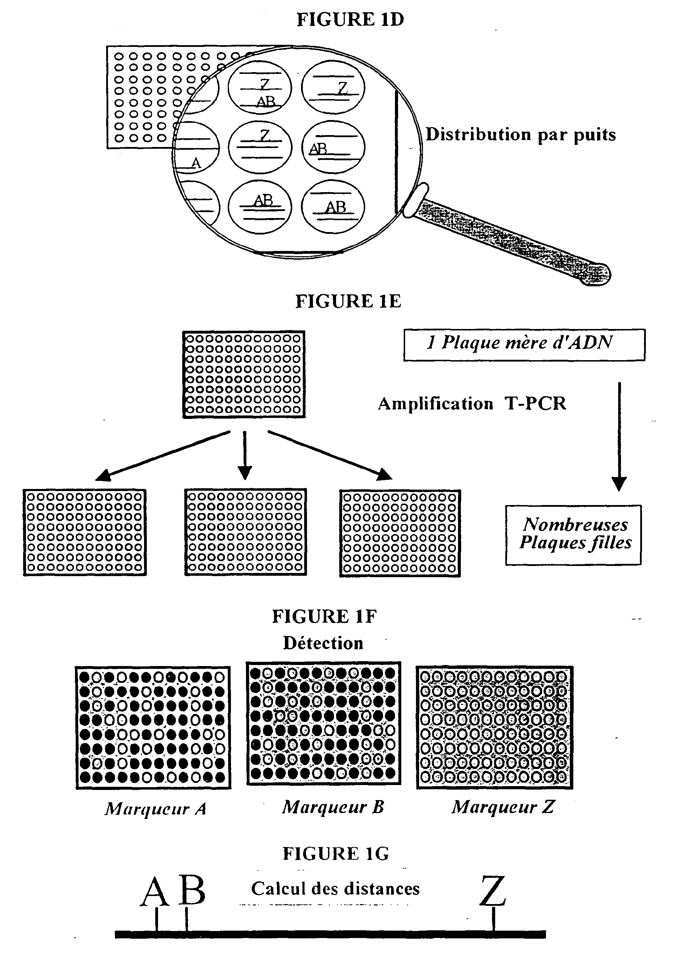

### Figure 1: Illustration du procédé selon l'invention

Trois marqueurs aléatoirement sélectionnés sur le génome sont représentés (A, B et Z), Différentes étapes de la cartographie Happy sont détaillées :
**(A)** L'ADN génomique est préparé à partir de cellules encapsulées dans l'agarose.
**(B)** fractionné aléatoirement,
**(C)** et les fragments sont sélectionnés à une taille donnée par électrophorèse en champs pulsés,
**(D)** puis déposés dans une plaque de microtitration à raison de 1 équivalent génome haploïde par puits.
**(E)** Ceci constituera le panel de cartographie après amplification par deux étapes de PCR.
**(F)** Chaque plaque fille est alors utilisée pour cartographier un marqueur. La PCR est effectuée à l'aide d'amorces spécifiques et l'analyse est réalisée ici sur gel d'agarose.
**(G)** La fréquence de co-ségrégation des marqueurs permet de déterminer leur position respective et d'établir une carte physique. Calcul de la fréquence d'association des marqueurs (LOD score) : La fréquence d'association des marqueurs dans un même puits dépend de la distance qui les sépare. Deux marqueurs proches sont souvent ou toujours présents ensemble (cas des marqueurs A et B) et deux marqueurs éloignés sont associés aléatoirement (cas des marqueurs A et Z ou B et Z).

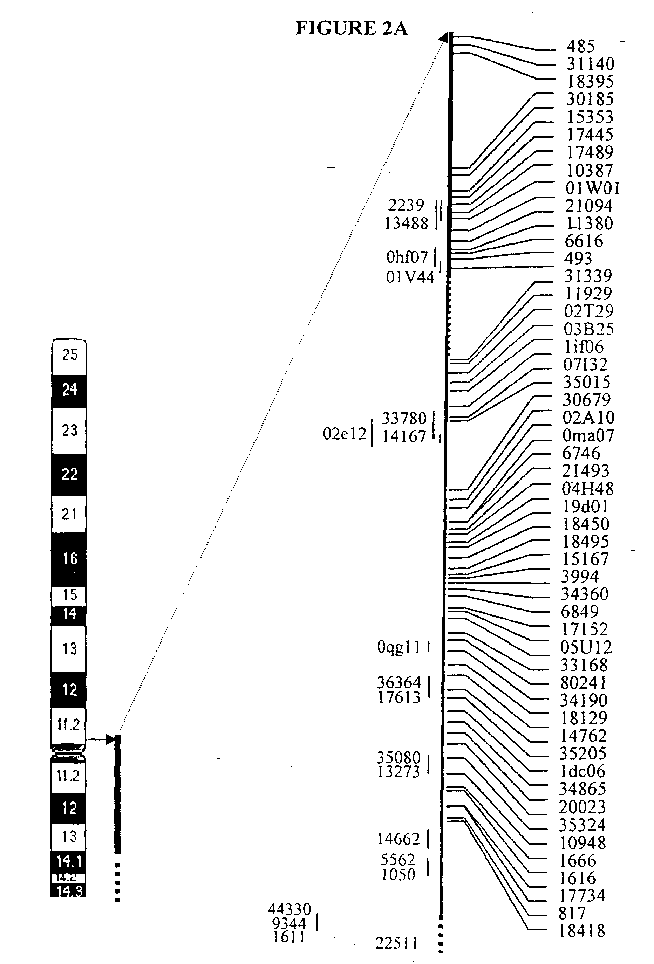

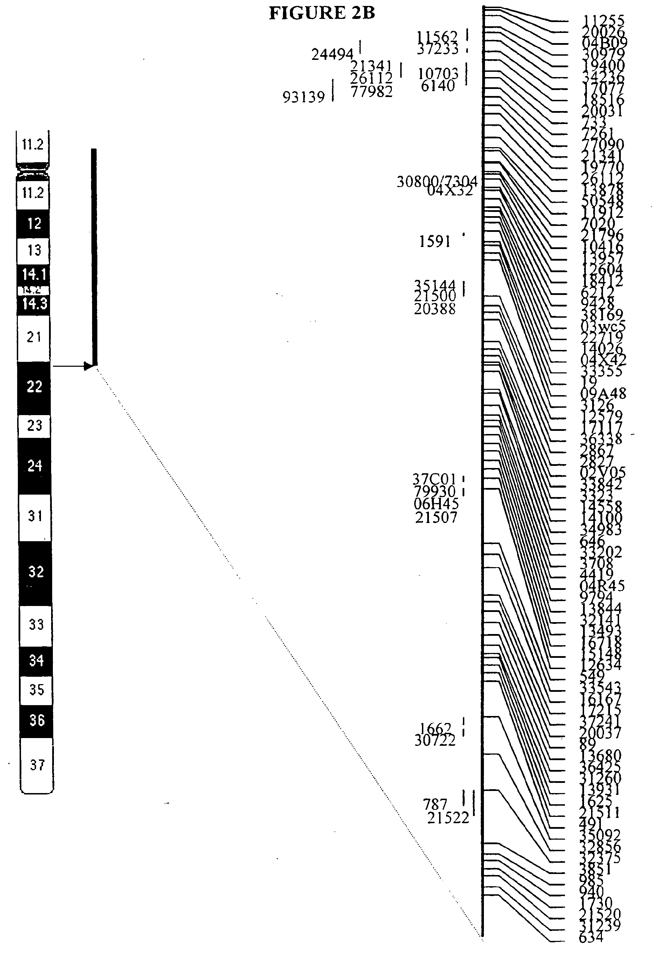

### Figure 2A et 2B: Résultats de la cartographie selon l'invention

La région du chromosome 2 humain est indiquée sur la gauche de la figure. La carte a été générée en associant les marqueurs deux à deux avec un LOD score supérieur à 4. Les marqueurs indiqués sur la partie droite de la carte ont été disposés sans ambiguïté les uns par rapport aux autres. Les marqueurs "flottants" repérés sur la partie gauche de la carte présentent une association forte (LOD score supérieur à 6) avec les marqueurs qui leur font face, mais ne présentent qu'une faible association avec les marqueurs adjacents. La localisation du centromère est indiquée par un trait pointillé.

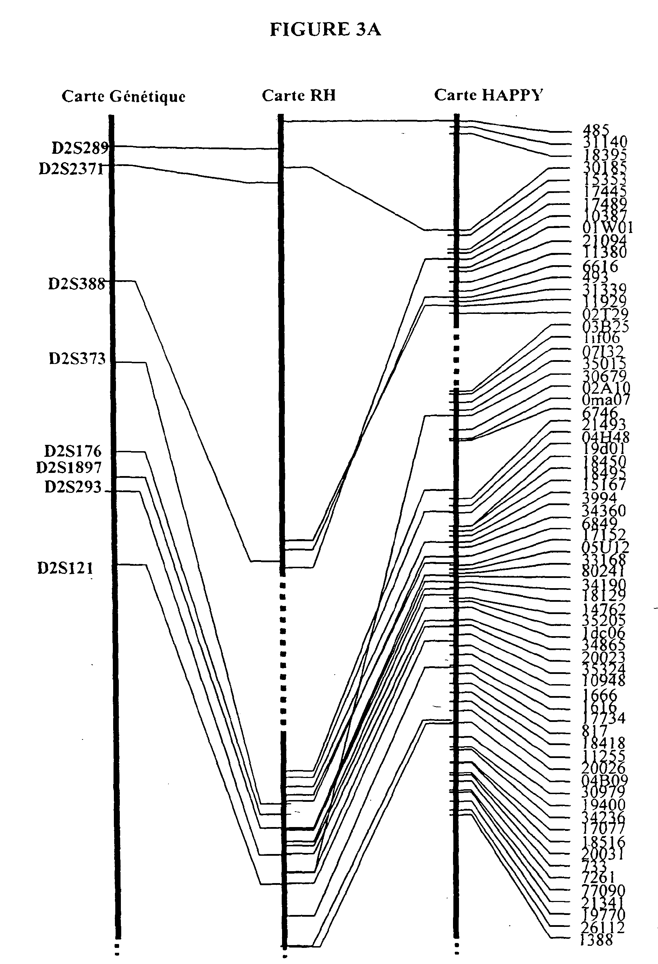

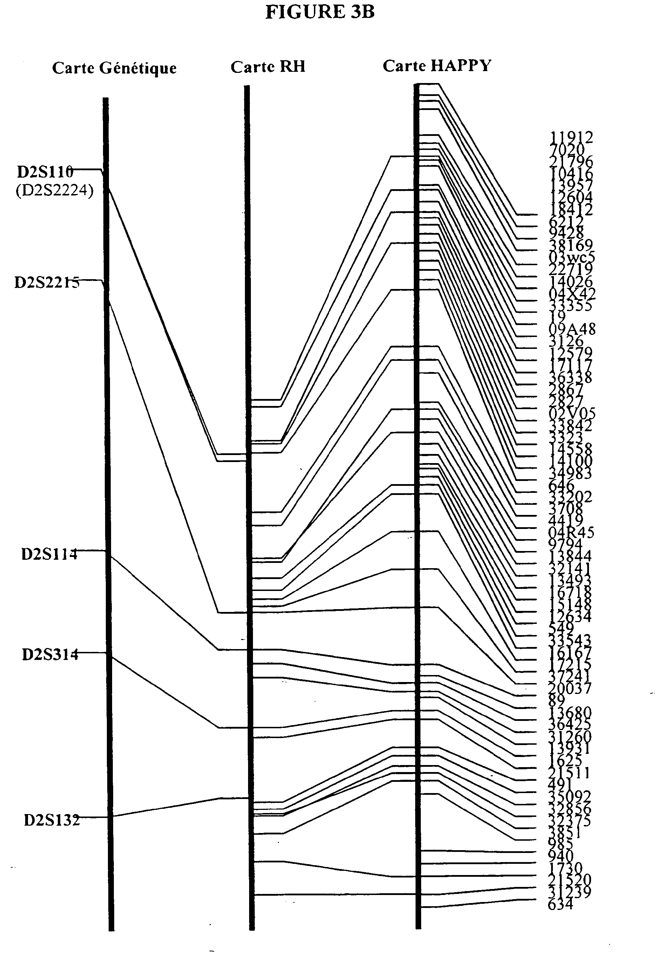

### Figure 3A et 3B: Comparaison des résultats de cartographie

Trois cartes sont représentées sur cette figure. La carte génétique (gauche) comporte les marqueurs polymorphes localisés par études familiales. La carte créée par hybrides d'irradiation (milieu) résulte de la localisation de marqueurs à la fois polymorphes et non polymorphes sur le panel G3 (Deloukas et al., 1998). La carte de droite a été obtenue grâce au procédé selon l'invention. Les marqueurs disposés sur deux cartes différentes sont représentés par des traits fins joignant les deux cartes. La localisation du centromère est indiquée par un trait pointillé.

La présente invention offre donc une nouvelle méthode pour aborder l'amplification *ad infinitum* d'une quantité faible d'ADN génomique - environ 1 équivalent génome haploïde, soit environ 2 pg dans le cas du génome humain-nécessaire à la constitution d'un panel utilisable pour la cartographie. La technique mise au point, réalisée en deux étapes de PCR permet une amplification de la quasi totalité du contenu génétique. Cette approche a permis de cartographier un nombre important de marqueurs sur une portion du chromosome 2 humain. Les résultats des cartographies effectuées en mettant en oeuvre le procédé selon l'invention sont en accord avec toutes les données décrites dans la littérature. Le procédé selon l'invention est simple à reproduire puisqu'il ne requiert que quelques jours voire quelques semaines. En outre, il est adaptable à n'importe quel niveau de résolution, selon la taille des fragments générés et sélectionnés. Il peut même aboutir au clonage moléculaire des fragments d'intérêt en vue d'un séquençage.

L'étape la plus critique dans la technique décrite est l'étape d'amplification totale et homogène du génome. La solution proposée par Paul Dear, inventeur de la méthode Happy Maping, est l'utilisation d'amorces ancrées sur des éléments répétés tout au long du génome (Alu, LINE) (IRS-PCR, pour Interspersed Repetitive Sequence-PCR). Cette solution a permis de positionner un millier de marqueurs sur le chromosome 14 humain (Dear *et al.,* 1998). Toutefois, l'IRS-PCR se limite à l'amplification et donc à la cartographie des marqueurs proches de séquences répétées et encadrés par elles. Ce type d'amplification est par conséquent restreint à une petite partie du génome (puisque seules les séquences encadrées par deux séquences alu ou LINE sont amplifiées) et ne s'applique qu'au génome humain ou de primates, à moins de définir des conditions d'amplifications spécifiques de chaque organisme étudié. C'est pourquoi, l'objet de la présente invention consiste en une technique permettant d'amplifier uniformément le génome, quel qu'il soit, sans perte de marqueur. Afin d'être utilisable pour la cartographie du type Happy Mapping, la méthode d'amplification doit être à la fois quantitative (amplifier à un niveau suffisant une quantité faible d'ADN initial correspondant à environ un équivalent génome haploïde) et qualitative (pas de perte notable de l'information génétique)

Comme précédemment évoqué, diverses approches ont été envisagées pour résoudre le problème. Les trois premières, issues de la littérature (Telenius *et al.,* 1992; Zhang *et al.,* 1992; Grothues *et al.,* 1993), n'ont apporté que peu de résultats. La méthode décrite par Telenius *et al* (1992) utilise un oligonucléotide dégénéré sur 6 positions pour une amplification par PCR réalisée en une seule étape (DOP-PCR, pour *Degenerated Oligonucleotide Primed-PCR*). Mais cette méthode ne fonctionne que si l'on dispose d'une grande quantité de matériel de départ (100 ng d'ADN génomique humain, soit 50000 équivalents génome haploïde environ ou 500 copies d'un chromosome isolé). Or, la cartographie du type Happy Mapping n'est possible qu'à partir d'échantillons d'ADN dont la quantité est voisine d'un équivalent génome haploïde. En utilisant les conditions décrites par Telenius *et al.* Il n'est pas possible d'obtenir une amplification de plus de 50% du matériel génétique initial. Ceci est en accord avec les résultats de Cheung et Nelson (1996) qui montrent que plus la quantité d'ADN génomique utilisée comme matrice est faible, en dessous d'un seuil de 600 pg -soit 300 équivalents génome haploïde dans le cas d'un génome de mammifère-, plus le risque de ne pas amplifier un locus est grand.

La seconde méthode décrite par Zhang *et al* (1992) utilise un oligonucléotide entièrement dégénéré sur ses 15 positions (PEP, Primer Extension Preamplification). En variant les conditions de PCR utilisées par Zhang *et al.* (nombre de cycles, températures d'hybridation, concentration en primer), il est apparu possible d'amplifier près de 90 % de l'ADN matrice à un minimum de 200 copies. Toutefois, cette approche ne permet pas de réamplification supplémentaire et limite donc grandement le nombre de marqueurs pouvant être cartographié. De plus, le niveau d'amplification ne permet pas une visualisation directe du produit d'amplification d'un marqueur donné mais oblige l'utilisation de deux couples d'amorces, le second étant niché dans le premier, et donc d'effectuer une amplification en deux étapes.

Une autre approche s'effectue en deux étapes et s'appuie sur un oligonucléotide dégénéré sur les 9 résidus situés dans sa partie 3' et comportant une séquence fixe en 5' utilisée pour la seconde étape d'amplification (T-PCR, pour *Tagged-PCR*) (Grothues *et al.,* 1993). Cependant, aucun aspect qualitatif de l'amplification n'est ici mis en évidence. Il a donc été nécessaire de déterminer si les paramètres quantitatifs et qualitatifs cruciaux seraient compatibles avec la cartographie Happy. En outre, il est clairement indiqué dans le brevet US 5,731,171 que la limite inférieure d'amplification est de 17 équivalents génome, ce qui n'est pas envisageable pour le Happy Mapping. Un autre inconvénient non négligeable dans la méthode décrite dans US 5,731.171 est le risque de contamination par de l'ADN étranger. En effet, lors de l'étape d'amplification à faible stringence, chaque cycle nécessite l'introduction 'manuelle' d'ADN polymérase. Le nombre de cycle devant être accru pour des quantités très faibles d'ADN, le risque de contamination s'en trouve d'autant augmenté.

L'ensemble de ces difficultés ont été écartées par la présente invention. Les recherches réalisées ont en effet permis d'utiliser une méthode du type PCR dans le cadre du Happy Mapping. La première étape d'amplification doit avantageusement respecter les paramètres tels que la concentration en primer, le nombre de cycles de PCR, la température d'hybridation, ainsi que la longueur de la chaîne dégénérée en 3'. Le choix de la séquence fixée en 5' de l'oligonucléotide est important pour la seconde étape d'amplification. Cette seconde phase est elle aussi très dépendante de la quantité de primer utilise et du nombre de cycles de PCR effectués.

### Procédé de cartographie comprenant une étape d'amplification ad infinitum.

Des cellules, récoltées chez le sujet à étudier sont encapsulées dans des blocs d'agarose (figure 1A), puis lysées afin de libérer l'ADN intact. Selon la taille des fragments désirés, l'ADN est ensuite cassée, ou non volontairement, de manière aléatoire (par irradiation γ, digestion enzymatique, ou par action mécanique) pour obtenir des fragments dont la taille dépend de la résolution choisie (figure 1B). L'ADN est alors migré par électrophorèse en champs pulsés (PFGE, pour *Pulsed Field Gel Electrophoresis*) pour séparer des fragments de taille homogène (figure 1C) qui sont répartis dans les 96 puits d'une plaque de microtitration de façon à n'avoir qu'une quantité voisine d'un équivalent génome haploïde d'ADN par puits (figure 1D). De cette façon, chaque puits a une représentation incomplète du génome qui lui est propre. L'intégralité de l'information génétique contenue dans chaque puits est alors amplifiée par PCR.

Chaque puits contient maintenant une grande quantité d'ADN ce qui permet de distribuer ce matériel dans de nombreuses plaques filles (figure 1E). Ainsi chaque plaque fille sera utilisée pour déterminer la distribution d'un marqueur ou d'un nombre limité de marqueur si la détection est faite par électrophorèse par exemple au moyen de la PCR en utilisant comme amorces des oligonucléotides spécifiques du marqueur en question (figure 1F). Les produits de PCR sont ensuite analysés en électrophorèse classique sur gel d'agarose. Cette opération est répétée pour un nombre élevé de marqueurs.

Dans le cas où un équivalent de génome est réparti par puits, chaque marqueur sera retrouvé, dans 65% des puits (loi de Poisson). Deux marqueurs non liés (séparés par une distance supérieure à la taille moyenne des fragments aliquotés) vont se distribuer de façon aléatoire et se retrouver ensemble dans 42% des puits (65% x 65%). A l'inverse, deux marqueurs séparés par une distance inférieure à cette taille moyenne ségrègeront d'autant plus fréquemment ensemble qu'ils seront proches l'un de l'autre, jusqu'à se retrouver ensemble dans 65% des aliquots dans le cas de marqueurs très étroitement liés. Chaque occurrence est enregistrée et les liaisons entre les marqueurs sont calculées (lod score) comme en génétique classique à l'aide de logiciels informatiques tels que RHmap (Boehnke *et al.,* 1991) ou RHmapper (Slonim *et al.,* 1995). La fréquence d'association entre deux marqueurs, traduisant la distance physique qui les sépare sur le chromosome, permet alors de déduire une carte regroupant tous les marqueurs associés, deux à deux, au sein d'un même groupe de liaison (figure 1G).

### Exemple I : Cartographie de marqueurs sur le chromosome 2 humain.

### Matériels et méthodes

### a) Préparation du panel de cartographie

Les lymphocytes humains sont prélevés puis purifiés sur Ficoll (Ficoll paque - Pharmacia) selon les instructions délivrées par le fournisseur. Après comptage, les cellules sont encapsulées dans des blocs d'agarose (InCert - FMC) à raison de 10⁵-10⁶ cellules/ml d'agarose, puis lysées *in situ* par 5 bains successifs. étalés sur 48 heures, dans 500 ml d'une solution de lyse (10mM Tris-HCl pH 7,4; 1mM EDTA; 1% LiDS) afin de libérer l'ADN intact. Les blocs d'agarose sont alors équilibrés dans la même solution dépourvue de LiDS. Au cours de ces étapes, l'ADN se fractionne aléatoirement de sorte que la taille moyenne des fragments résultants avoisine les 6-7 Mb (résultats non montrés). L'ADN est alors migré par électrophorèse en champs pulsés (PFGE, pour *Pulsed Field Gel Electrophoresis*) dans un CHEF Mapper (BioRad) [gel d'agarose 0,8% (SeaKem Gold agarose - FMC) dans du TAE 1X; migration à 2V/cm dans du TAE 1X durant 96 heures @ 14°C; temps d'inversion de champs de 40 min 23 sec à 106°] [3]. Après 48 heures de migration; les puits ainsi que les 2 mm supérieurs du gel sont éliminés afin d'éviter toute contamination par des petits fragments d'ADN qui seraient emprisonnés dans les puits et relargués progressivement au cours de l'électrophorèse. A la fin de la migration, la partie du gel contenant les marqueurs de taille (chromosomes de S. pombe - FMC) est colorée au BET. L'ADN génomique est alors prélevé dans le gel à l'aide de microcapillaires en verre (Minicaps 10µl - Hirschmann Laborgerate) puis ces échantillons d'agarose sont répartis dans les 96 puits d'une plaque de microtitration de façon à n'avoir que 2 pg d'ADN par puits, soit un peu moins d'un équivalent de génome haploïde [4]. De cette façon, chaque puits a une représentation incomplète du génome qui lui est propre.

La première étape consiste en la détermination de la quantité d'ADN présent dans les puits et le nécessaire ajustement à environ 2pg pour du matériel humain. Pour cette analyse, un marqueur a été aléatoirement choisi sur chaque chromosome humain pour déterminer la quantité d'ADN à distribuer dans la plaque mère. Si l'on part d'un équivalent génome haploïde, chaque marqueur doit être présent dans 65% des puits analysés, d'après la loi statistique de Poisson (Dear *et al.,* 1993), ce qui est vérifié par PCR nichée.

### b) Amplification aléatoire de l'ADN génomique

Le contenu en ADN de chaque puits est amplifié en deux étapes par PCR. La première phase consiste en une amplification utilisant un seul oligonucléotide (T3N6 5' AATTAACCCTCACTAAAGGGNNNNNN 3') (SEQ ID n°1). La réaction est effectuée dans un volume final de 50µl, par puits de la microplaque, contenant 50mM KCl; 10mM Tris-HCl pH 8,3; 0,001% (w/v) gélatine; 2,5mM MgCl2; 200µM dNTP (Pharmacia); 5 unités d'AmpliTaq polymérase (Perkin Elmer) et 4µM d'oligonucléotide T3N6. Chaque essai est recouvert d'une goutte d'huile minérale et la réaction de PCR est effectuée dans un thermocycleur PTC-200 (MJ Research) 1 x [5 min @*.*95°C]; 50x [45 sec @92°C, 2 min @37°C, 37°C-55°C 0,1°C/sec, 4 min @55°C]; 15x [45 sec @92°C, 1 min @55°C, 3 min @72°C]; 1x [5 min @72°C].

Pour la seconde phase de la réaction, 1/50e de la première phase est utilisé comme matrice. La réaction de PCR est effectuée dans un volume réactionnel final de 10µl, par puits de la microplaque, contenant 50mM KCl; 10mM Tris-HCl pH 9; 0,1% Triton® X-100; 2,5mM MgCl2; 300µM dNTP (Pharmacia); 2 unités de Taq DNA polymérase (Promega) et 1,5µM d'oligonucléotide T3 (5' AATTAACCCTCACTAAAGGG 3') (SEQ ID n°2). La réaction de PCR est effectuée dans un thermocycleur PTC-200 (MJ Research): 1x [2 min @94°C]; 50x [30 sec @92°C, 45 sec @54°C, 3 min @72°C]; 1x [5 min @72°C]. Cette seconde phase est effectuée quatre fois de manière indépendante, puis les quatre réactions sont mélangées.

### c) Création d'une carte d'une portion du chromosome 2 humain

### Matériels et méthodes

### Analyse de la distribution des marqueurs

Des marqueurs de type EST (*Expressed Sequence Tag*) ainsi que quelques marqueurs de type microsatellites déjà cartographiés ont été sélectionnés dans les banques de données suivantes sur le chromosome 2 humain :
http://www.ncbi.nlm.nih.gov/genemap
http://www.ncbi.nlm.nih.gov/SCIENCE96/:
http://www-shgc.stanford.edu/Mappin/rh/MapsV2/search2html

La distribution des marqueurs dans chaque plaque fille est analysée par PCR à partir d'un microlitre de la seconde phase d'amplification. Le milieu réactionnel contient 50mM de KCl; 10mM de Tris-HCl pH9,0; 0,1 % de Triton X-100; 2,5mM de MgCl2; 250µM de chaque dNTP; 1µM des deux oligonucléotides amont et aval spécifiques de chaque marqueur analysé et 0,5U de Taq DNA polymerase (Promega) dans 10µl. La réaction comprend 1 premier cycle de dénaturation d' 1 min à 92°C, suivi de 20 cycles de 20s à 92°C, 30s à 60°C avec un décrément de température de 0.5°C/cycle et 30s à 72°C, puis 10 cycles de 20s à 92°C, 30s à 50°C et 30s à 72°C ainsi qu'1 cycle d'achèvement de l'élongation de 2mn à 72°C.

Après migration sur gel d'agarose 3%, les produits de PCR sont visualisés et photographiés sur une plaque UV équipée d'une caméra CCD (Bioprint).

### Traitement informatique

Par la suite, la présence/absence de chaque marqueur dans les puits des microplaques est saisie manuellement sur ordinateur sous la forme d'un nombre trinaire comportant les chiffres 0 (absence du marqueur), 1 (présence du marqueur) ou 2 (résultat douteux), interprétable par les logiciels de cartographie classique. Les différents groupes de liaison sont ensuite formés, après les calculs de fréquence d'association des marqueurs (LOD score), à l'aide du programme RH2PT de l'ensemble des logiciels RHMAP (Boehnke *et al*., 1991). Les distances séparant les marqueurs ainsi que leur ordre sont enfin déterminés à l'aide du programme RHMAXLIK de ce même ensemble.

### Résultats

Dans un premier temps, 190 marqueurs humains ont été sélectionnés dans les banques de données sur une zone couvrant environ 60 Mégabases du chromosome 2 humain. Chacun de ces marqueurs a été testé par PCR sur les puits de la microplaque constituant le panel de cartographie. La présence/absence de ces marqueurs est visualisée par PCR suivie d'une électrophorèse et coloration au BET. Parmi ces 190 marqueurs, 176 ont été intégrés à la carte (> 92%; figure 2). Les autres, en raison d'un taux de rétention s'éloignant trop de la norme, ont été écartés. En effet, seuls sont gardés les marqueurs dont le nombre de positifs est de p ± 2√ (p - [p²/n]) où p est le nombre moyen de positifs par marqueur et n le nombre d'aliquots constituant le panel. L'analyse statistique de la répartition des marqueurs s'est effectuée à l'aide de la suite logicielle RHMap (Boehnke *et al.,* 1991). Dans un premier temps, un premier regroupement de marqueurs a été opéré par une analyse 'deux à deux' en fixant un LOD score seuil minimal de 8 pour visualiser les associations entre marqueurs. Les marqueurs ont été placés par rapport à leurs voisins par analyse multipoint au sein de chaque groupe de liaison généré à un LOD score de 8. Par la suite, les groupes ont été disposés les uns par rapport aux autres en choisissant un LOD score de 4.

Parmi les 176 marqueurs ainsi cartographiés, 133 se sont placés sans ambiguïté les uns par apport aux autres (>75%; figure 2): Toutefois, 43 autres marqueurs (partie gauche de la carte; figure 2), montrant une association suffisamment forte avec un ou plusieurs marqueurs déjà localisés pour être acceptée comme réelle, n'ont pas pu être placés de manière formelle en raison d'une moindre association avec les marqueurs situés dans l'entourage immédiat. Pour cette raison, ils ont été indiqués par des barres flottantes. Il est nécessaire de noter que si la carte a été réalisée avec 176 marqueurs (densité de 1 marqueur/340 kb), des résultats similaires auraient été obtenus en n'utilisant que 75 marqueurs judicieusement choisis ce qui porte la densité minimale à 1 marqueur/800 kb).

### Exemple 2 : Comparaison de la carte HAPPY avec les autres cartes disponibles dans les banques de données

Afin de valider la carte mentionnées ci-dessus, les données obtenues ont été comparées avec celles qui sont disponibles dans les banques informatiques. Actuellement, de nombreuses données issues de la cartographie par hybrides d'irradiation ou par études familiales sont disponibles. La comparaison de nos résultats avec ceux obtenus par hybrides d'irradiation sur le panel de cartographie G3 qui a été obtenu d'une irradiation cellulaire à 10000 Rad (Stewart et al, 1997) a été choisie en priorité. La taille moyenne des fragments obtenus est d'environ 4 Mb, ce qui est très proche de la taille de fragments que nous avons sélectionné pour la constitution de notre panel (5,5 Mb). 48 marqueurs communs ont été placés sur les deux cartes (figure 3). L'ordre général de ces marqueurs est respecté dans ces deux cartes, puisque seules 4 inversions ont été mises en évidence. Ces inversions deux à deux ne sont observées que sur des marqueurs proches sur le génome à une distance estimée inférieure à 500 kb à comparer à la limite de résolution du panel (http://www-shgc.stanford.edu) rh estimée à 300 kb. Dans la région située immédiatement au-dessus du centromère, trois marqueurs présentent une position relativement divergente. Ceci peut être mis en relation avec le fait que la cartographie par hybrides d'irradiation peut entraîner des biais dans la résolution des régions entourant le centromère. Ce fait peut aussi expliquer les différences de distances séparant les marqueurs dans ces régions (figure 3). La position des marqueurs que nous avons obtenue est bien en accord avec la carte génétique disponible dans les banques de données (figure 3).

### REFERENCES

Apostolakos (1993) Anal. Biochem., 213, 277-284
Bcmdt (1995) Anal. Biochem., 225, 252-257
Boehnke M et al. (1991) Statistical methods for multipoint radiation hybrid Mapping. Am J Hum Genet, 49, 1174-1188
Cheung VG et Nelson SF (1996) Whole genome amplification using a degenerate oligonucleotide primer allows hundreds of genotypes to be performed on less than one nanogram of genomic DNA. Proc. Natl. Acad. Sci. USA, 93, 14676-14679
Cox DR et al. (1990) Radiation hybrid Mapping: a somatic cell genetic method for constructing high-resolution maps of mammalian chromosomes. Science, 250, 245-250
Dear PH et Cook PR (1993) Happy Mapping: linkage Mapping using a physical analogue of meiosis. Nucl Acids Res, 21, 13-20
Dear PH et al (1998) A high resolution metric HAPPY map of human chromosome 14. Genomics, 48, 232-241
Deloukas P et al. (1998) A Physical Map of 30,000 Human Genes. Science, 282, 744-746
Grothues D et al (1993) PCR amplification of megabase DNA with tagged random primers (T-PCR). Nucleic Acids Res, 21, 1321-1322
Gyapay G et al. (1996) A radiation hybrid map of the human genome, Hum Mol Genet. 5, 339-346
Kawai (1993) Anal. Biochem., 209, 63-69
Lander ES et Botstein DB (1989) Mapping mendelian factors underlying quantitative traits using RFLP linkage maps. Genetics, 121, 185-199
Piper MB et al., (1998) A HAPPY map of Crypto sporidium parvum. Genome research, 8, 1299-1307
Running (1990) BioTechniques, 8, 276-277
Slonim D et al. (1995) RHMAPPER: An interactive computer package for constructing radiation hybrid maps. Cold Spring Harbor Meeting on Genome Mapping and Sequencing. May 10-14.
Stewart EA et al. (1997) An STS-based radiation hybrid map of the human genome. Genome Res, 7, 422-433
Telenius H et al. (1992) Degenerate Oligo-Primed PCR: General amplification of target DNA by a single degenerate primer. Genomics, 13, 718-725
Venter et al. (1998) Science compass 5 juin, p. 1540.
Weber JL et Myers EW (1997) Human whole-genome shotgun sequencing. Genome Res, 7, 401-409
Zhang L et al (1992) Whole genome amplification from a single cell: Implications for genetic analysis. Proc. Natl. Acad. Sci. USA, 89, 5847-5851

## Revendications

1. Procédé de détection de marqueurs d'une molécule d'ADN **caractérisé en ce qu'**il comprend les étapes consistant à :
a) Casser la molécule d'ADN de sorte à obtenir des fragments d'ADN dont la taille dépend de la résolution choisie,
b) répartir lesdits fragments dans des réceptacles de façon à avoir une quantité d'ADN comprise entre environ 0,5 à 1,5 équivalent génome haploïde d'ADN par réceptacle,
c) amplifier l'ADN contenu dans les réceptacles par une méthode d'amplification comprenant les étapes suivantes : i) Une première amplification à l'aide d'une amorce comprenant de 10 à 30 nucléotides définis en son extrémité 5', et de 5 à 10 nucléotides aléatoires en son extrémité 3', ii) une deuxième amplification à l'aide d'une amorce comprenant au moins les oligonucléotides définis de l'extrémité 5' de l'amorce utilisée à l'étape i).
d) détecter la présence ou l'absence de marqueurs dans les réceptacles.

2. Procédé selon.la revendication 1 **caractérisé en ce que** l'amorce utilisée à l'étape i) comprend 20 nucléotides définis en son extrémité 5', et 6 nucléotides aléatoires en son extrémité 3'.

3. Procédé selon la revendication 2 **caractérisé en ce que** l'amorce utilisée à l'étape i) correspond à la séquence SEQ ID n°1.

4. Procédé selon la revendication 1 **caractérisé en ce que** l'amorce utilisée à l'étape ii) comprend les 20 nucléotides définis en l'extrémité 5' de l'amorce utilisée à l'étape i).

5. Procédé selon la revendication 4 **caractérisé en ce que** l'amorce utilisée à l'étape ii) correspond à la séquence SEQ ID n°2.

6. Procédé selon l'une des revendications 1 à 5 **caractérisé en ce que** ladite molécule d'ADN correspond ou provient d'un génome, d'un chromosome, ou d'un fragment d'un génome ou d'un chromosome.

7. Procédé selon la revendication 6 **caractérisé en ce que** la molécule d'ADN est extraite à partir de cellules encapsulées dans des blocs d'agarose puis lysées afin de libérer ladite molécule intacte.

8. Procédé selon la revendication 7 **caractérisé en ce que** lesdites cellules sont des cellules provenant de toute cellule notamment, mais pas exclusivement, végétale et animale.

9. Procédé selon l'une des revendications 1 à 7 **caractérisé en ce que** la molécule d'ADN est cassé si nécessaire par irradiation γ, par digestion enzymatique, et/ou par action mécanique.

10. Procédé selon l'une des revendications 1 à 9 **caractérisé en ce que** les fragments d'ADN sont séparés de préférence par électrophorèse en champs pulsés pour obtenir des fragments de taille homogènes avant la répartition (étape b)).

11. Procédé selon l'une des revendications 1 à 10 **caractérisé en ce que** les fragments sont répartis dans les puits d'une plaque de microtitration à 96, 182, ou 384 puits, de préférence de sorte à avoir environ 1 équivalent génome haploïde par puits.

12. Procédé selon l'une des revendications 1 à 11 **caractérisé en ce que** l'intégralité de l'information génétique contenu dans les puits est amplifiée.

13. Procédé selon l'une des revendications 1 à 12 **caractérisé en ce que** l'ADN amplifié dans chaque puits est distribué de manière à préparer des plaques filles.

14. Procédé selon l'une des revendications 1 à 13 **caractérisé en ce que** les marqueurs sont détectés dans les puits des plaques filles.

15. Procédé selon l'une des revendications 1 à 14 **caractérisé en ce que** les marqueurs susceptibles d'être présent dans les puits sont amplifiés à l'aide d'amorces spécifiques avant l'étape de détection.

16. Procédé selon l'une des revendications 1 à 15 **caractérisé en ce que** la détection s'effectue directement après électrophorèse ou à l'aide de sondes spécifiques des marqueurs.

17. Procédé selon la revendication 16 **caractérisé en ce que** lesdites sondes sont des sondes de captures immobilisées directement ou indirectement sur un support solide.

18. Procédé selon l'une des revendications 16 et 17 **caractérisé en ce** les sondes sont marquées au moyen d'un marqueur choisi notamment parmi les isotopes radioactifs, les enzymes susceptibles d'agir sur un substrat chromogène, fluorigène ou luminescent, et des composés chimiques chromophores, des composés chromogènes, fluorigènes et luminescents.

19. Procédé selon l'une des revendications 1 à 18 **caractérisé en ce que** la détection s'effectue sur des puces à ADN.

20. Utilisation du procédé selon l'une des revendications 1 à 19 pour l'identification de gènes conférant un phénotype d'intérêt.

21. Utilisation selon la revendication 20 pour l'identification de gènes responsable de maladies héréditaires, notamment chez les humains.

22. Utilisation selon la revendication 21 pour l'identification de QTL (Quantitative Trait Loci).

23. Utilisation selon la revendication 20 pour l'identification de gènes conférant un phénotype d'intérêt chez les plantes ou les animaux.

24. Utilisation du procédé selon l'une des revendications 1 à 19 pour une aide à la reconstruction de shotguns massifs destiné au séquençage d'une molécule d'ADN.

## Claims

1. A method for detecting markers of a DNA molecule **characterized in that** it consists of the steps:
a) breaking the DNA molecule in order to obtain DNA fragments, the size of which depends on the selected resolution,
b) distributing said fragments in receptacles in order to have an amount of DNA between about 0.5 to 1.5 DNA haploid genome equivalent per receptacle,
c) amplifying the DNA contained in the receptacles by an amplification method comprising the following steps: i) a first amplification by means of a primer comprising 10 to 30 defined nucleotides at its 5' end and 5 to 10 random nucleotides at its 3' end, ii) a second amplification by means of a primer comprising at least the defined oligonucleotides of the 5' end of the primer used in step i),
d) detecting the presence or absence of markers in the receptacles.

2. The method according to claim 1, **characterized in that** the primer used in step i) comprises 20 defined nucleotides at its 5' end and 6 random nucleotides at its 3' end.

3. The method according to claim 2, **characterized in that** the primer used in step i) corresponds to sequence SEQ ID no.1.

4. The method according to claim 1, **characterized in that** the primer used in step ii) comprises the 20 defined nucleotides at the 5' end of the primer used in step i).

5. The method according to claim 4, **characterized in that** the primer used in step ii) corresponds to sequence SEQ ID no.2.

6. The method according to any of claims 1 to 5, **characterized in that** said DNA molecule corresponds to or is derived from a genome, a chromosome, or a genome or chromosome fragment.

7. The method according to claim 6, **characterized in that** the DNA molecule is extracted from encapsulated cells in agarose blocks, then lyzed in order to release said intact molecule.

8. The method according to claim 7, **characterized in that** said cells are cells derived from any cell, notably but not exclusively any plant and animal cell.

9. The method according to any of claims 1 to 7, **characterized in that** the DNA molecule is broken, if necessary, by γ irradiation, enzymatic digestion, and/or by mechanical action.

10. The method according to any of claims 1 to 9, **characterized in that** the DNA fragments are preferably separated by pulsed field gel electrophoresis in order to obtain fragments of homogeneous sizes before distribution (step b).

11. The method according to any of claims 1 to 10, **characterized in that** the fragments are distributed in wells of a 96-, 182- or 384-well microtitration plate, preferably in order to have about 1 haploid genome equivalent per well.

12. The method according to any of claims 1 to 11, **characterized in that** the entire genetic information contained in the wells is amplified.

13. The method according to any of claims 1 to 12, **characterized in that** the amplified DNA in each well is distributed so as to prepare daughter plates.

14. The method according to any of claims 1 to 13, **characterized in that** the markers are detected in the wells of the daughter plates.

15. The method according to any of claims 1 to 14, **characterized in that** the markers likely to be present in the wells are amplified by means of specific primers before the detection step.

16. The method according to any of claims 1 to 15, **characterized in that** the detection is directly carried out after electrophoresis or by means of probes specific to the markers.

17. The method according to claim 16, **characterized in that** said probes are capture probes directly or indirectly immobilized on a solid support.

18. The method according to any of claims 16 and 17, **characterized in that** the probes are marked by means of a marker notably selected from radioactive isotopes, enzymes capable of acting on a chromogenic, fluorigenic or luminescent substrate, and chromophore chemical compounds, chromogenic, fluorigenic and luminescent compounds.

19. The method according to any of claims 1 to 18, **characterized in that** the detection is performed on DNA chips.

20. The use of the method according to any of claims 1 to 19 for identifying genes imparting a phenotype of interest.

21. The use according to claim 20 for identifying genes responsible for hereditary diseases, notably in humans.

22. The use according to claim 21 for identifying QTLs (Qantitative Trait Loci).

23. The use according to claim 20 for identifying genes imparting a phenotype of interest in plants or animals.

24. The use of the method according to any of claims 1 to 19 for helping the reconstruction of massive shotguns for sequencing a DNA molecule.

## Patentansprüche

1. Verfahren zum Nachweis von Markern eines DNA-Moleküls, **dadurch gekennzeichnet, dass** es die Schritte umfasst, welche daraus bestehen:
a) das DNA-Molekül derart zu schneiden, dass DNA-Fragmente erhalten werden, deren Größe von der gewählten Auflösung abhängt,
b) die Fragmente in Behälter derart zu verteilen, dass eine DNA-Menge zwischen etwa 0,5 und 1,5 Äquivalenten eines haploiden DNA-Genoms pro Behälter vorliegt,
c) die in den Behältern enthaltene DNA zu amplifizieren durch eine Amplifizierungsmethode, welche die folgenden Schritte umfasst: i) eine erste Amplifizierung mit Hilfe eines Primers, welcher definierte 10 bis 30 Nukleotide an seinem 5'-Ende und 5 bis 10 zufällige Nukleotide an seinem 3'-Ende umfasst, ii) eine zweite Amplifizierung mit Hilfe eines Primers, welcher wenigstens die definierten Oligonukleotide des 5'-Endes des in Schritt i) eingesetzten Primers umfasst,
d) das Vorhandensein oder Fehlen von Markern in den Behältern nachzuweisen.

2. Verfahren nach Anspruch 1, **dadurch gekennzeichnet, dass** der in Schritt i) eingesetzte Primer 20 definierte Nukleotide an seinem 5'-Ende und 6 zufällige Nukleotide an seinem 3'-Ende umfasst.

3. Verfahren nach Anspruch 2, **dadurch gekennzeichnet, dass** der in Schritt i) eingesetzte Primer der Sequenz SEQ ID NO:1 entspricht.

4. Verfahren nach Anspruch 1, **dadurch gekennzeichnet, dass** der in Schritt ii) eingesetzte Primer die 20 definierten Nukleotide am 5'-Ende des in Schritt i) eingesetzten Primers umfasst.

5. Verfahren nach Anspruch 4, **dadurch gekennzeichnet, dass** der in Schritt ii) eingesetzte Primer der Sequenz SEQ ID NO:2 entspricht.

6. Verfahren nach einem der Ansprüche 1 bis 5, **dadurch gekennzeichnet, dass** das DNA-Molekül einem Genom, einem Chromosom oder einem Fragment eines Genoms oder eines Chromosoms entspricht oder von einem Genom, einem Chromosom oder einem Fragment eines Genoms oder eines Chromosoms abstammt.

7. Verfahren nach Anspruch 6, **dadurch gekennzeichnet, dass** das DNA-Molekül aus Zellen extrahiert wird, welche in Agarose-Blöcken eingeschlossen, dann lysiert wurden, um das intakte Molekül freizusetzen.

8. Verfahren nach Anspruch 7, **dadurch gekennzeichnet, dass** die Zellen Zellen sind, die von einer jeglichen, insbesondere, aber nicht ausschließlich pflanzlichen und tierischen Zelle stammen.

9. Verfahren nach einem der Ansprüche 1 bis 7, **dadurch gekennzeichnet, dass** das DNA-Molekül, sofern erforderlich, durch γ-Bestrahlung, durch enzymatischen Verdau und/oder durch mechanische Einwirkung geschnitten wird.

10. Verfahren nach einem der Ansprüche 1 bis 9, **dadurch gekennzeichnet, dass** die DNA-Fragmente vorzugsweise durch Pulsfeldgelelektrophorese aufgetrennt werden, um vor dem Verteilen (Schritt b)) Fragmente von homogenen Größen zu erhalten.

11. Verfahren nach einem der Ansprüche 1 bis 10, **dadurch gekennzeichnet, dass** die Fragmente in den Vertiefungen einer Mikrotiterplatte mit 96, 182 oder 384 Vertiefungen verteilt werden vorzugsweise derart, dass ungefähr 1 Äquivalent.eines haploides Genom pro Vertiefung vorliegt.

12. Verfahren nach einem der Ansprüche 1 bis 11, **dadurch gekennzeichnet, dass** die Gesamtheit der genetischen Information, die in den Vertiefungen enthalten ist, amplifiziert wird.

13. Verfahren nach einem der Ansprüche 1 bis 12, **dadurch gekennzeichnet, dass** die amplifizierte DNA in jeder Vertiefung derart verteilt wird, dass Tochterplatten hergestellt werden.

14. Verfahren nach einem der Ansprüche 1 bis 13, **dadurch gekennzeichnet, dass** die Marker in den Vertiefungen der Tochterplatten nachgewiesen werden.

15. Verfahren nach einem der Ansprüche 1 bis 14, **dadurch gekennzeichnet, dass** die Marker, die in den Vertiefungen vorhanden sein können, vor dem Nachweisschritt mit Hilfe von spezifischen Primem amplifiziert werden.

16. Verfahren nach einem der Ansprüche 1 bis 15, **dadurch gekennzeichnet, dass** der Nachweis direkt nach einer Elektrophorese oder mit Hilfe von für die Marker spezifischen Sonden erfolgt.

17. Verfahren nach Anspruch 16, **dadurch gekennzeichnet, dass** die Sonden Einfang-Sonden, die direkt oder indirekt auf einem festen Träger immobilisiert sind, sind.

18. Verfahren nach einem der Ansprüche 16 und 17, **dadurch gekennzeichnet, dass** die Sonden mittels eines Markers, welcher insbesondere unter den radioaktiven Isotopen, den Enzymen, die auf ein chromogenes, fluorogenes oder lumineszierendes Substrat einwirken können, und chemischen chromophoren Verbindungen, chromogenen, fluorogenen und lumineszierenden Verbindungen ausgewählt wird, markiert sind.

19. Verfahren nach einem der Ansprüche 1 bis 18, **dadurch gekennzeichnet, dass** der Nachweis auf DNA-Chips erfolgt.

20. Verwendung des Verfahrens nach einem der Ansprüche 1 bis 19 zur Identifizierung von Genen, welche einen Phänotyp von Interesse verleihen.

21. Verwendung nach Anspruch 20 zur Identifizierung von Genen, welche für Erbkrankheiten, insbesondere beim Menschen, verantwortlich sind.

22. Verwendung nach Anspruch 21 für die Identifizierung von QTL (Quantitative Trait Loci).

23. Verwendung nach Anspruch 20 für die Identifizierung von Genen, welche einen Phänotyp von Interesse verleihen, bei Pflanzen oder Tieren.

24. Verwendung des Verfahrens nach einem der Ansprüche 1 bis 19 für ein Hilfsmittel bei der Rekonstruktion von massiven Shotguns, welche für die Sequenzierung eines DNA-Moleküls bestimmt ist.


### LISTAGE DE SEQUENCE

<110> CNRS (CENTRE NATIONAL DE LA RECHERCHE SCIENTIFIQUE
<120> PROCEDE DE CARTOGRAPHIE D'UNE MOLECULE D'ADN COMPRENANT UNE ETAPE D'AMPLIFICATION AD INFINITUM
<130> D18074
<160> 2
<170> Patent In Vers. 2.0
<210> 1
   <211> 26
   <212> ADN
   <213> Artificial Séquence
<220>
   <223> Amorce i (N représente n'importe quel nucléotide)
<400> 1
   aattaaccct cactaaaggg nnnnnn 26
<210> 2
   <211> 20
   <212> ADN
   <213> Artificial Sequence
<220>
   <223> Amorce ii
<400> 2
   aattaaccct cactaaaggg 20